# EUROPEAN PATENT SPECIFICATION

(11) **EP 2 632 545 B1**
(45) Date of publication and mention of the grant of the patent: **23.11.2016**
(21) Application number: 11770026.0
(22) Date of filing: 05.10.2011
(51) Int. Cl.: A61N 1/372

(54) **AN EXTERNAL CONTROLLER FOR AN IMPLANTABLE MEDICAL DEVICE FORMED USING A SUB-ASSEMBLY**
EXTERNE STEUERUNG FÜR EINE IMPLANTIERBARE MEDIZINISCHE VORRICHTUNG MIT EINER BAUGRUPPE
UNITÉ DE COMMANDE EXTERNE POUR DISPOSITIF MÉDICAL IMPLANTABLE FORMÉE À L'AIDE D'UN SOUS-ENSEMBLE

(30) Priority: 25.10.2010 US 406341 P
(43) Date of publication of application: 04.09.2013
(73) Proprietor: Boston Scientific Neuromodulation Corporation, Valencia, CA 91355 (US)
(72) Inventor: AGHASSIAN, Daniel, Glendale, CA 91208 (US); BUNYAN, Navin, Noel, Valencia, CA 91354 (US); FUNDERBURK, Jeffrey, V., Stevenson Ranch, CA 91381 (US); DRONOV, Vasily, San Jose, CA 95125 (US)
(74) Representative: Vossius & Partner Patentanwälte Rechtsanwälte mbB
(86) International application number: PCT/US2011/054880
(87) International publication number: WO 2012/057977

(56) References cited:
- WO-A1-2008/014022
- WO-A2-03/022357
- US-A1- 2005 075 685
- US-A1- 2005 131 479
- US-A1- 2009 118 796

## Description

### FIELD OF THE INVENTION

The present invention relates to the design and packaging of an external telemetry device having particular applicability to implantable medical device systems.

### BACKGROUND

Implantable stimulation devices are devices that generate and deliver electrical stimuli to body nerves and tissues for the therapy of various biological disorders. Examples of such devices include pacemakers for treating cardiac arrhythmia, defibrillators for treating cardiac fibrillation, cochlear stimulators for treating deafness, retinal stimulators for treating blindness, muscle stimulators for producing coordinated limb movement, spinal cord stimulators for treating chronic pain, cortical and deep brain stimulators for treating motor and psychological disorders, and other neural stimulators for treating urinary incontinence, sleep apnea, shoulder sublaxation and the like. The present invention may find applicability in all such applications, although the description that follows will generally focus on the use of the invention within a Spinal Cord Stimulation (SCS) system, such as that disclosed in U.S. Patent 6,516,227.

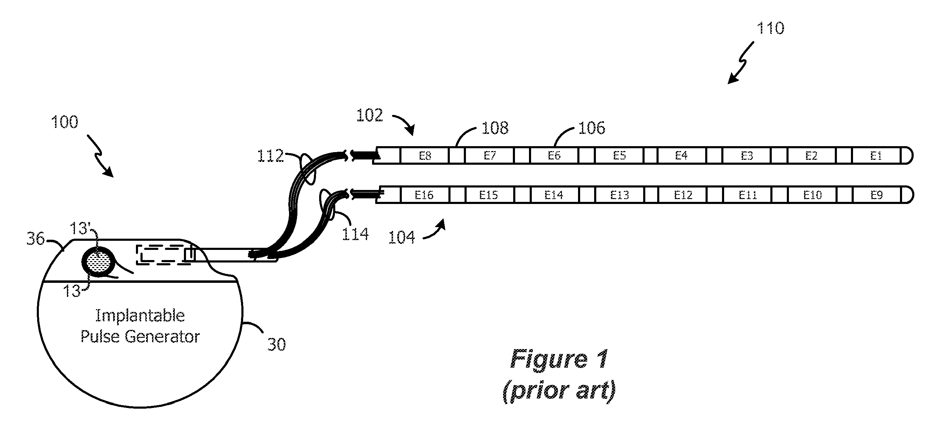

Spinal cord stimulation is a well-accepted clinical method for reducing pain in certain populations of patients. As shown in FIG. 1, a SCS system typically includes an Implantable Pulse Generator (IPG) 100, which includes a biocompatible case 30 formed of titanium for example. The case 30 typically holds the circuitry and power source or battery necessary for the IPG to function, although IPGs can also be powered via external RF energy and without a battery. The IPG 100 is coupled to electrodes 106 via one or more electrode leads (two such leads 102 and 104 are shown), such that the electrodes 106 form an electrode array 110. The electrodes 106 are carried on a flexible body 108, which also houses the individual signal wires 112 and 114 coupled to each electrode. In the illustrated embodiment, there are eight electrodes on lead 102, labeled E1-E8, and eight electrodes on lead 104, labeled E9-E16. However, the number of leads and electrodes is application specific and therefore can vary.

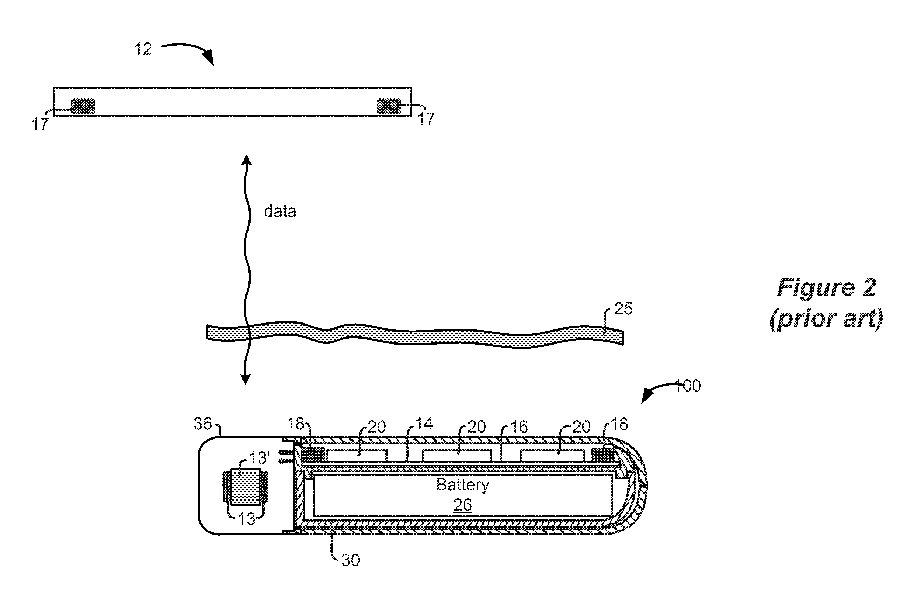

Portions of an IPG system are shown in FIG. 2 in cross section, and include the IPG 100 and an external controller 12. The IPG 100 typically includes an electronic substrate assembly 14 including a printed circuit board (PCB) 16, along with various electronic components 20, such as microprocessors, integrated circuits, and capacitors mounted to the PCB 16. Two coils are generally present in the IPG 100, a telemetry coil 13 used to transmit/receive data to/from the external controller 12, and a charging coil 18 for charging or recharging the IPG's power source or battery 26 using an external charger (not shown). The telemetry coil 13 can be mounted within the header connector 36 as shown, or can be housed within the case 30.

As just noted, an external controller 12, such as a hand-held programmer or a clinician's programmer, is used to wirelessly send data to and receive data from the IPG 100. For example, the external controller 12 can send programming data to the IPG 100 to set the therapy the IPG 100 will provide to the patient. Also, the external controller 12 can act as a receiver of data from the IPG 100, such as various data reporting on the IPG's status.

The communication of data to and from the external controller 12 occurs via magnetic inductive coupling. When data is to be sent from the external controller 12 to the IPG 100 for example, coil 17 is energized with an alternating current (AC). Such energizing of the coil 17 to transfer data can occur using a Frequency Shift Keying (FSK) protocol, for example, such as disclosed in U.S. Patent Application Publication 2009/0024179. Energizing the coil 17 induces an electromagnetic field, which in turn induces a current in the IPG's telemetry coil 13, which current can then be demodulated to recover the original data. Communication from the IPG 100 to the external controller 12 occurs in essentially the same way. As is well known, inductive transmission of data or power occurs transcutaneously, i.e., through the patient's tissue 25, making it particularly useful in a medical implantable device system.

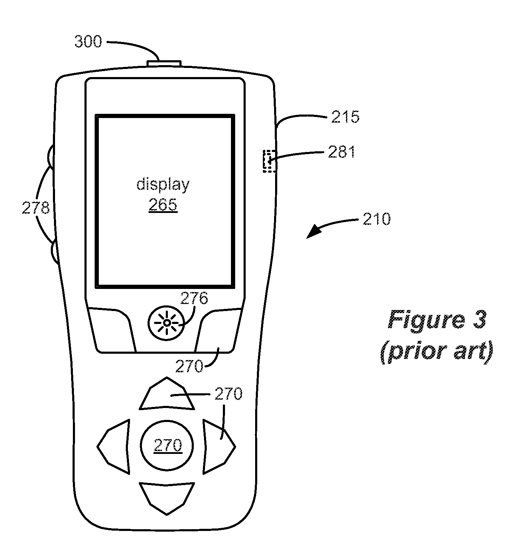

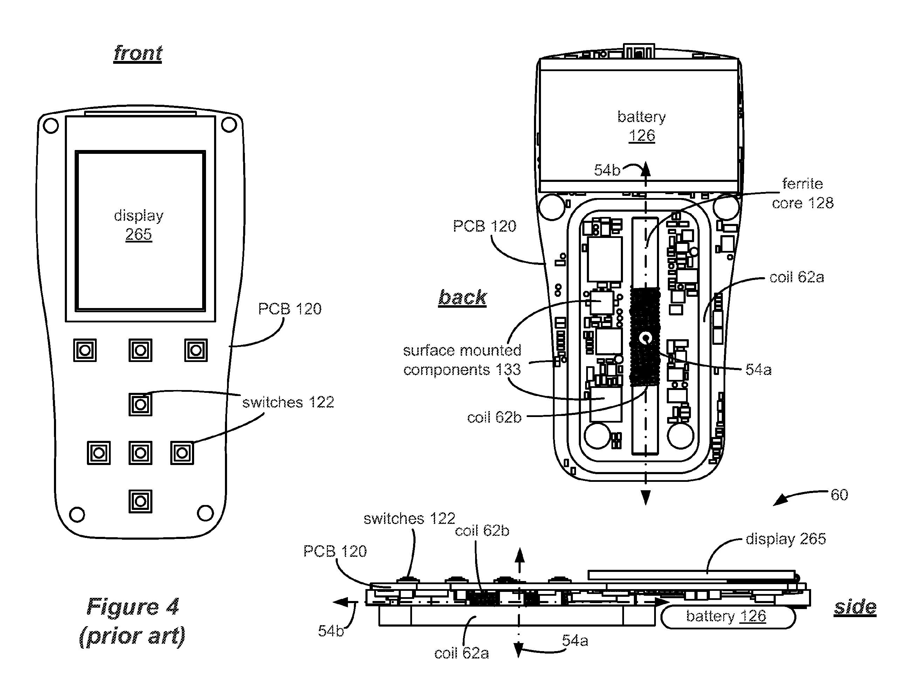

An external controller 210 disclosed in U.S. Patent Publication 2009/0118796 is shown in FIG. 3 (a different external controller is disclosed in WO 2008/014022 A1). This external controller 210 may integrate the functions of an external controller and an external charger that wirelessly provides power to the IPG's battery, but such battery charging aspects are not shown in FIG. 3 for simplicity. External controller 210 comprises a user interface, which is somewhat similar to a cell phone or to other external controllers used in the art, in that it includes a display 265 and various buttons 270. Soft key buttons 278 can be used to select various functions, which functions will vary depending on the status of the menu options available at any given time. A speaker is also included within the housing of the external controller to provide audio cues to the user (not shown). Alternatively, a vibration motor can provide feedback for users with hearing impairments or others who would like to turn the audio cues off. Unlock button 281, recessed into the side of the housing, can be used to unlock the buttons, if they become automatically or manually locked. External controller 210 also has a USB port 300, which can connect to a charging coil (as discussed in detail in the '796 Publication), to a computer, or to a power source (a wall outlet) to either power the external controller directly or to charge its battery 126 (FIG. 4).

The user interface generally allows the user to telemeter data (such as a new therapy program) from the external controller 210 to the IPG 100, or to monitor various forms of status feedback from the IPG. The software in the controller 210 (preferably implemented as microcode accessible by the controller 210's microcontroller) accordingly provides logical menu options to the display 265. For example, when the controller is first turned on, the display 265 may provide selectable options for the user to either program the IPG 100 or to change stimulation settings.

The internal structure of external controller 210, with its cover 215 removed, is shown in FIG. 4. As shown, a printed circuit board (PCB) 120 is central to the internal construction of the controller 210. The front side of the PCB 120 carries aspects of the user interface, including the display 265 and pressure-sensitive switches 122 for receiving presses to the various user interface buttons 270 (FIG. 3). The external controller 210 controls data telemetry by energizing at least one coil 62a or 62b. The telemetry coils 62a and 62b and a battery 126 along with other integrated and discrete components necessary to implement the functionality of the external controller are located on the back side of the PCB 120. The battery 126 can comprise a Lithium (Li)-ion battery or a Li-ion polymer battery for example.

As seen in the back and side views of FIG. 4, the two telemetry coils 62a and 62b are respectively wrapped around axes 54a and 54b which are orthogonal. Coil 62a is wrapped in a racetrack configuration around the back of the PCB 120, while coil 62b is wrapped around a ferrite core 128. Further discussion of the benefits of orthogonally-oriented telemetry coils 62a and 62b can be found in U.S. Patent Publication 2009/0069869, which with the reader is assumed familiar. Briefly, when used to transmit data, the two coils 62a, 62b are driven (for example, with FSK-modulated data) out of phase, preferably at 90 degrees out of phase. This produces a magnetic field which rotates, and which reduces nulls in the coupling between the external controller 210 and the telemetry coil 13 in the IPG 100. Should dual coils 62a and 62b also receive status transmissions from the IPG 100, the two coils are used in conjunction with receiver circuitry which likewise phase shifts the received modulated data signals from each coil and presents their sum to typical demodulation circuitry.

After they are wound, the telemetry coils 62a and 62b are soldered to the PCB 120, and the coils 62a and 62b (including ferrite core 128 around which coil 62b is wound) must then be secured in place, usually by affixing them to the PCB using epoxy. These are cumbersome steps in the manufacturing process, and are prone to failure or reliability problems. Soldering and epoxying the coils 62a and 62b must be accomplished by hand, after the other various components 133 (a few of which are shown in FIG. 4) have already been surface mounted to the PCB 120. This risks damaging these other components 133. Additionally, extra care must be taken to well affix coil 62b to the PCB 120, in particular because of the fragile nature of the ferrite core 128, which is made of material having high magnetic permeability such as MnZn or NiZn and is prone to fracture. Even after the ferrite core 128 is secured, it is still generally exposed and protrudes from the PCB 120. Thus, the ferrite core 128 can be easily damaged, should it be bumped during remaining steps in the manufacturing process.

The external controller's battery 126 has similar manufacturing concerns. It too must be secured and soldered to the PCB 120 in some fashion, which can likewise damage the various components 133 previously surface mounted to the PCB. And like the coils 62a and 62b and the ferrite core 128, the battery 126 protrudes from the PCB 120 and is susceptible to damage.

In addition to these problems, the mechanical design of the external controller 210 has another disadvantage in that the telemetry coils 62a and 62b, the ferrite core 128, and the battery 126 cover a large portion of the surface area of the back of PCB 120. The various other components 133 surface mounted to the PCB 120 should not be placed underneath such areas where the coils 62a and 62b, the ferrite core 128, and the battery 126 will be affixed, and thus space on the PCB 120 must be reserved for these additional structures. Reserving space on the PCB 120 for these additional structures limits where the other components 133 can be surface mounted, and can complicate routing and layout of the PCB 120. Reserving space on the PCB 120 for the coils 62a and 62b, the ferrite core 128, and the battery 126 also generally means that the PCB 120 will need to be larger than would otherwise be necessary if the PCB 120 were only to accommodate the components 133.

The inventors of the present invention recognize the existence of these problems in prior art external controllers and provide solutions to these problems in this disclosure.

### BRIEF DESCRIPTION OF THE DRAWINGS

FIG. 1 shows an implantable pulse generator (IPG), and the manner in which an electrode array is coupled to the IPG, in accordance with the prior art.
FIG. 2 shows wireless communication of data between an external controller and an IPG, in accordance with the prior art.
FIG. 3 shows an external controller, in accordance with the prior art.
FIG. 4 shows the internal components of the external controller of FIG. 3, in accordance with the prior art.
FIGS. 5A-5B show exploded bottom and top views, respectively, of internal components of an improved external controller, in accordance with an embodiment of the present invention.
FIG. 6A-6C show back, top, and side views, respectively, of a sub-assembly used in the manufacture of the improved external controller of FIGS. 5A-5B, in accordance with an embodiment of the present invention.
FIG. 7 shows a perspective view of the improved external controller upon completion of its manufacture.

### DETAILED DESCRIPTION

The description that follows relates to use of the invention within a spinal cord stimulation (SCS) system. However, the invention is not so limited. Rather, the invention may be used with any type of implantable medical device system that could benefit from an improved structural design of a data telemetry device used to communicate with an implanted device. For example, the present invention may be used as part of a system employing an implantable sensor, an implantable pump, a pacemaker, a defibrillator, a cochlear stimulator, a retinal stimulator, a stimulator configured to produce coordinated limb movement, a cortical and deep brain stimulator, or in any other neural stimulator configured to treat any of a variety of conditions.

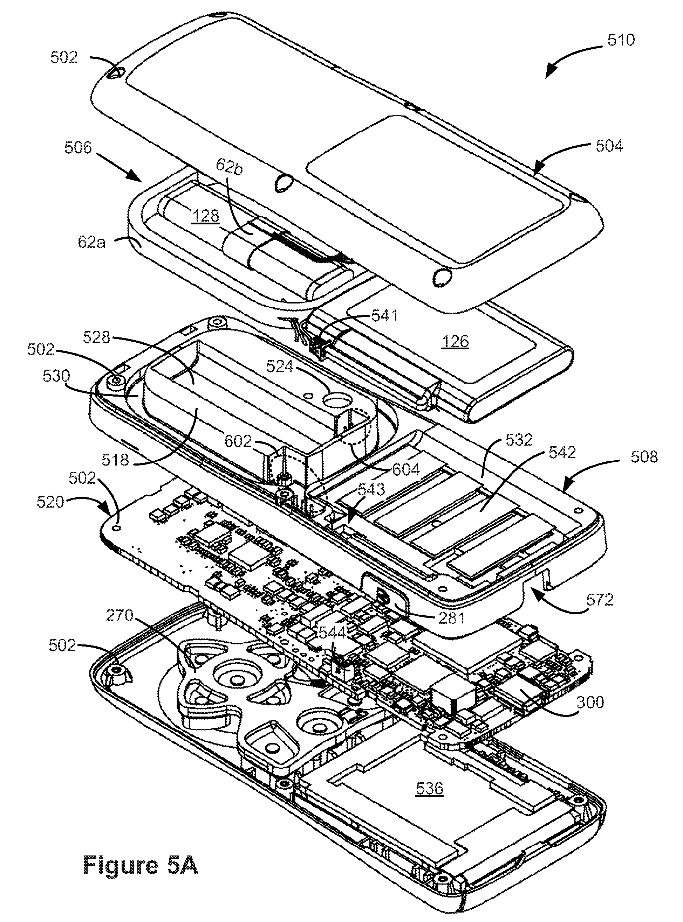

An improved external controller 510 is illustrated in FIGS. 5A-5B, which offers significant improvements in design and manufacturing over prior art external controllers such as the external controller 210 (FIGS. 3 and 4) discussed in the Background. To the extent that similar structures in the external controller 210 are used in the improved external controller 510, they retain the same element numerals, and are not again described for simplicity.

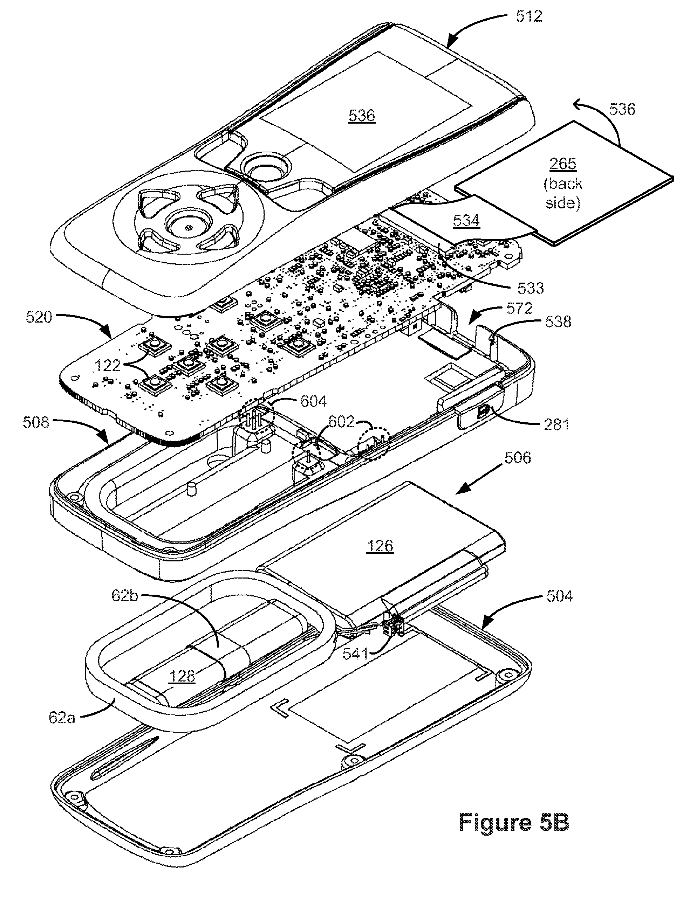

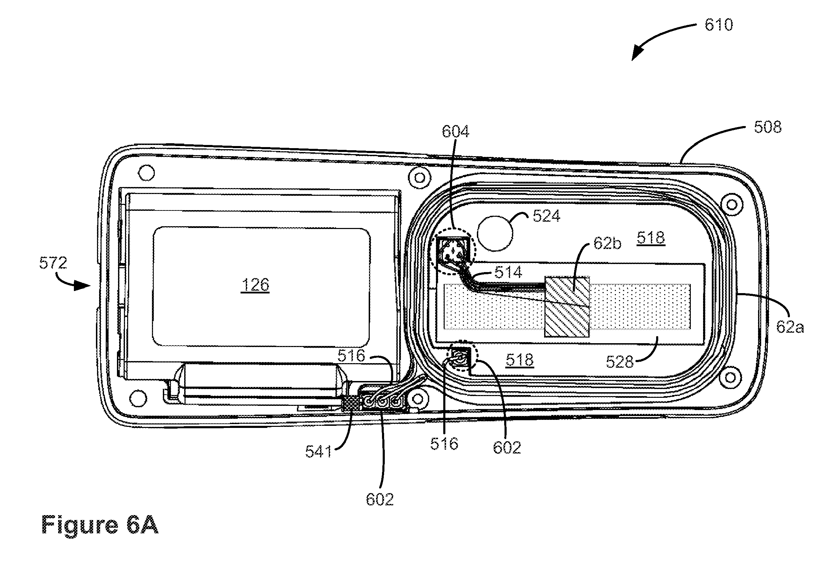

FIGS. 5A and 5B provide exploded bottom and top views, respectively, of the main parts that comprise the external controller 510, including a back cover 504, a front cover 512, an electronics chassis 508, a printed circuit board (PCB) 520, and battery/coil components 506. The battery/coil components 506 include the coils 62a and 62b, the ferrite core 128, and the battery 126. Two coils 62a and 62b are preferred so that the external controller 510 can benefit from the technique disclosed in the '869 Publication discussed in the Background. This however is not strictly necessary and the external controller 510 can comprise only one communication coil, or more than two. The battery/coil components 506 and the PCB 520 will be incorporated into the electronics chassis 508 during manufacturing, resulting in a sub-assembly 610, which will be discussed further with respect to FIGS. 6A-6C.

It should be noted that, while FIGS. 5A and 5B show only one PCB 520, it is possible to include more than one PCB in the external controller 510. For example, instead of the PCB 520, the external controller can include two smaller PCBs. In some embodiments, one PCB can be placed on one side of the electronics chassis 508 and another PCB on the other side. In other embodiments, a second PCB can be attached to the PCB that is incorporated into the electronics chassis. Showing one PCB 520 is merely exemplary.

The front cover 512, back cover 504, and the electronics chassis 508 are preferably comprised of mold-injected plastic. The electronics chassis 508 incorporates pins 602 and 604 protruding from both its front and back sides, which will be connected to the coils 62a and 62b and to the PCB 520 during manufacturing, as will be explained in detail later. The front cover 512 includes a clear display window 536 though which the display 265 can be seen. As shown in FIG. 5B, the display 265 couples to a connector 533 on the front side of the PCB 520 via a flexible bus 534. (The display 265 is not shown in FIG. 5A for simplicity). The flexible bus 534 allows the display 265 to be bent over as indicated by arrow 536 so that its front side shows through the display window 536. The front cover 512 has one or more mechanical features (not shown) that ensure the display 265 is property aligned with and securely attached to the display window 536. The buttons 270 comprise a single rubber gasket as shown in FIG. 5A, which meet with various switches 122 on the front side of the PCB 520. (The buttons 270 are not shown in FIG. 5A for simplicity). One or more holes (not shown) in the front cover 512, back cover 504, or the PCB 520 can align with a speaker (not shown) to facilitate transmission of audible indicators. A USB port 300 is coupled to the back side of the PCB 520, and is made accessible through a hole 572 in the side of the chassis 508.

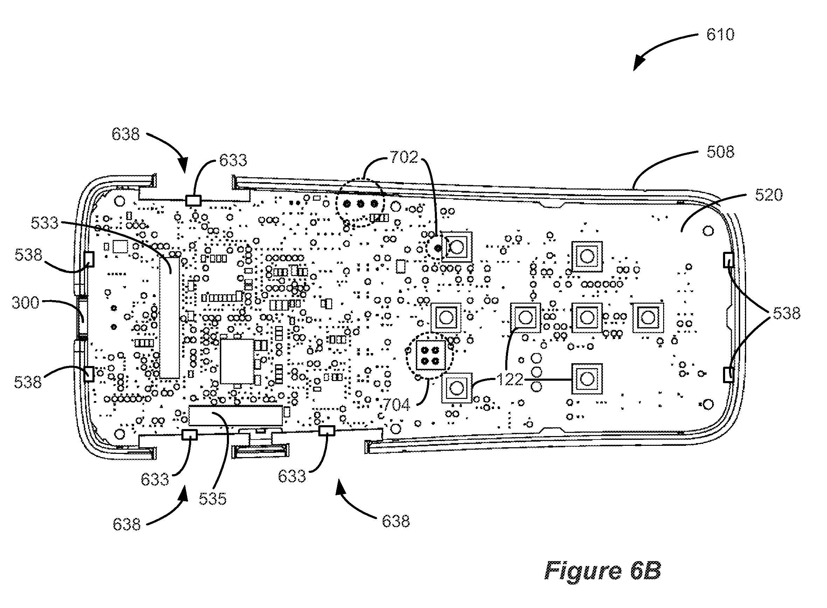

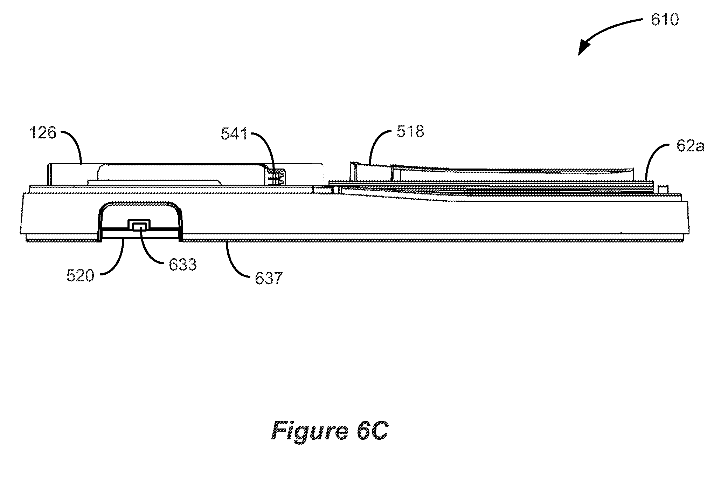

As noted earlier, the battery/coil components 506 and the PCB 520 will be incorporated into the electronics chassis 508 during manufacturing, resulting in a sub-assembly 610, which is shown in FIGS. 6A, 6B, and 6C in back, front, and side views, respectively. The electronics chassis 508 offers a secure and durable structure on which the coils 62a and 62b, the ferrite core 128, and the battery 126 can be placed, and additionally will form the sides of the external controller 510 once manufacturing is completed.

Manufacture of the sub-assembly 610 occurs as follows. First, the pre-wound coils 62a and 62b are coupled to the back side of the electronics chassis 508. To assist in placement of the coils 62a and 62b, and as best seen in FIG. 5A, the chassis 508 is formed with a protrusion 518 inside of which a pocket 528 is formed for housing telemetry coil 62b and the ferrite core 128 around which it is wrapped. The pocket 528 is made with a shape substantially corresponding to the shape of the wound coil 62b/ferrite core 128 combination. This correspondence in shape enables this combination to fit snuggly into the pocket 528, which prevents it from moving. After the ferrite and telemetry coil 62b are placed in the pocket 528, as shown in FIG. 6A, the pocket 528 can be filled with epoxy (not shown) to further limit movement to protect the coil 62b and the fragile ferrite core 128. Even if the ferrite core 128 is thereafter cracked, for example, because the external controller 510 is dropped, the epoxy will hold the cracked pieces in place, and the ferrite core 128 will still function adequately and will not damage other components. Because the pocket 528 is also suitably deep, the coil 62b and ferrite core 128 are recessed below the top of the protrusion 518 (see the side view of FIG. 6C), which prevents them from being damaged in subsequent manufacturing steps.

Protrusion 518 also creates a recess 530 around it, as best seen in FIG 5A. Recess 530 is used for easy and secure placement of coil 62a, as shown in FIG. 6A. Once coil 62a is placed into recess 530, it too can be epoxied in place. Although not shown, the walls of the recess 530 can be made high enough such that the coils 62a does not protrude from the recess 530, although providing such complete protection is of lesser concern for the ferrite-less coil 62a.

After the coils 62a and 62b have been coupled to the electronics chassis 508, they can be electrically connected to pins 602 and 604 respectively, as best shown in FIG. 6A. As mentioned earlier, these pins 602 and 604 are ultimately used to connect the coils 62a and 62b to the PCB 520, as will be discussed later. Coil 62b has loose ends 514 which are soldered to pins 604, and coil 62a has loose ends 516 which are soldered to pins 602. Such soldering can occur by hand, or can be automated. As best shown in FIG. 5A, the pins 602 and 604 are recessed within the chassis 508 so that they do not protrude above its top (e.g., above the protrusion 518). (See also the side view of FIG. 6C, which does not show protrusion of the pins 602 or 604). Recessing the pins 602 and 604 protects them and their connection to the loose ends 514 and 516 from mechanical damage. Pins 602 and 604 can be incorporated into the chassis 508 when it is mold injected, or can be passed through the chassis 508 after the coils 62a and 62b are soldered to them.

As shown, coil 62a has four loose ends 516, and coil 62b has four loose ends 514, and accordingly there are four pins 602 and four pins 604 to receive these loose ends, respectively. This is because, in the illustrated embodiment, each of coils 62a and 62b actually comprises two separate windings to benefit from the technique described in U.S. Patent Publication 2009/0024179, with which the reader is assumed familiar. Briefly, the '179 Publication describes how a communication coil in an external controller can be split into two parts. The secondary winding can be coupled to the battery 126 via a diode, such that when the primary winding of the coil is resonating, some of the energy is shunted off and directed back to the battery 126. However, use of the technique of the '179 Publication is not required, and instead, the coil 62a could comprise two loose ends 516, the coil 62b could comprise two loose ends 514, and the chassis 508 could accordingly comprises only two pins 602 and two pins 604.

After connection of the coils 62a and 62b to the electronics chassis 508, the battery 126 is next connected to the back side of the chassis. As best seen in FIG. 5A, the chassis 508 is formed with an indentation 532 which is roughly shaped to match the curved edges of the battery 126. To hold the battery 126 within the indentation 532, the bottom of the indentation is lined with an adhesive, such as strips of double-sided foam tape 542. Tape 542 adequately holds the battery 126, and provides some cushion to protect the battery 126 from mechanical damage. However, the battery 126 could also be affixed to the chassis 508 in other well-known ways. Although not shown, the walls of the indentation 532 could be made high enough such that the battery 126 would not protrude above those walls, thus completely recessing the battery 126 for maximum protection.

While pocket 528, recess 530, and indentation 532 have each been given separate names for clarity, it should be understood that each can be considered generically as a "recess" for purposes of this disclosure.

After the coils 62a and 62b and the battery 126 have been affixed to the electronics chassis 508, the PCB 520 is affixed to the chassis 508. The PCB 520 is at this point essentially fully formed using traditional surface mounting technology. Most of the components 133 (FIG. 4) are mounted to the back side of the PCB 520 (i.e., that which faces the chassis 508), but some components such as the switches 122 and the display connector 533 are mounted to the front side of the PCB 520, as shown in FIG. 6B. The front side of PCB 520 can also contain a test connector 535 for testing the sub-assembly 610 once it is completed, and before enclosure of the sub-assembly between the front cover 512 and the back cover 504.

The PCB 520 is positioned into the front side of the chassis 508, and as shown in FIG. 6B, the PCB 520 is shaped to match the contours of the chassis 508 to ensure a snug fit. To further assist in securing the PCB 520 within the chassis 508, clamps 538 are used, which clamps 538 were formed into the chassis 508 when it was mold injected. The PCB 520 can be "clicked" inside of the clamps 538 to achieve a secure fit. Notice in FIG. 6C that the PCB 520 is slightly recessed from the top edge 637 of the chassis 508. This protects the edges of the PCB 520, and also assists in protecting any components 133 (FIG. 4) mounted to the front side of the PCB 520 from mechanical damage.

As shown in FIG. 6B, the PCB is formed with through-holes 702 and 704. When the PCB 520 is secured within the chassis 508, these through-holes 702 and 704 are respectively penetrated by the pins 602 and 604 on the front side of the chassis 508. By way of review, these pins 602 and 604 were earlier coupled to the coils 62a and 62b on the back side of the chassis 508. When the pins 602 and 604 protrude through the holes 702 and 704, they are then soldered, for example, by hand or in an automated fashion. This couples the coils 62a and 62b to appropriate circuit nodes on the PCB 520, i.e., to nodes meeting with the telemetry circuitry on the PCB 520.

Thereafter, the battery 126 is coupled to the PCB 520. The battery 126 is formed with a connector 541. The chassis 508 is formed with a hole 543, and the PCB 520 includes a connector 544 designed to mate with the battery's connector 541, as best shown in FIG. 5A. When the PCB 520 is positioned within the chassis 508, the hole 543 and PCB connector 544 align, such that the battery connector 541 can mate to the PCB connector 544, as shown in FIG. 6A, thereby coupling the battery 126 to appropriate circuitry nodes on the PCB 520. As this point, the sub-assembly 610 is fully manufactured, and can be tested (e.g., using connector 535) if necessary.

Before continuing with remaining steps in the assembly of the external controller 510, some benefits provided by the sub-assembly 610 can be appreciated in contradistinction to the prior art external controller of FIGS. 3 and 4. The sub-assembly 610 comprises a mechanically stable unit which is easy to manufacture, and which is also easy to manipulate during subsequent manufacturing steps. The coils 62a and 62b, and in particular the fragile ferrite core 128, are protected and secured by virtue of being recessed and affixed within the electronic chassis 508, and the battery 126 is similarly protected although perhaps to a lesser degree if not completely recessed. Additionally, the coils 62a and 62b are not directly soldered to the PCB 520, but instead are connected to the PCB 520 by the electronic chassis 508 (or more specifically, pins 602 and 604) as an intermediary. This is a much simpler and more reliable way of connecting the coils 62a and 62b to the PCB 520, as this connection only involves soldering of the pins 602 and 604 to the PCB 520 as opposed to the otherwise fragile loose ends 514 and 516 of the coils. Moreover, note that the chassis 508 generally lies in a different parallel plane from the PCB 520. This leaves room between the PCB 520 and the chassis 508 for components 133 (FIG. 4) to be placed on the PCB 520, even in spaces underlying the coils 62a and 62b and the battery 126. As such, no space has to be reserved on the PCB 520 for these additional structures, and the components 133 can therefore be more densely packed and their routing on the PCB facilitated. Obviating the need for additional space also means the PCB 520 can be made smaller if desired.

Once the sub-assembly 610 is completed, manufacture of the external controller can be completed. Holes 638 are provided in the side of the chassis 508 and the PCB 520 to accommodate plastic soft key buttons 278 and an unlock button 281 (FIG. 3), which plastic buttons are slid into position where they interface with switches 633 (FIGS. 6B and 6C) extending from the edges of the PCB 520. Positioning buttons 278 and 281 in this fashion ultimately integrates them into the edges of the external controller 510. The gasket comprising the buttons 270 is placed over the switches 122. The display 265 is coupled to the connector 533 (FIG. 5A), flipped over as discussed previously, and if necessary affixed to the front cover 512. Then, the front cover 512 and the back cover 504 are positioned in place around the sub-assembly 610, and all of the components are bolted into place. In this regard, FIG. 5A shows that the front cover 512, chassis 508, PCB 520, and the back cover 504 all comprise aligned bolt holes 502. While only one such set of bolt holes 502 is labeled in FIG. 5A, one will notice that six such sets appear around the periphery of these components.

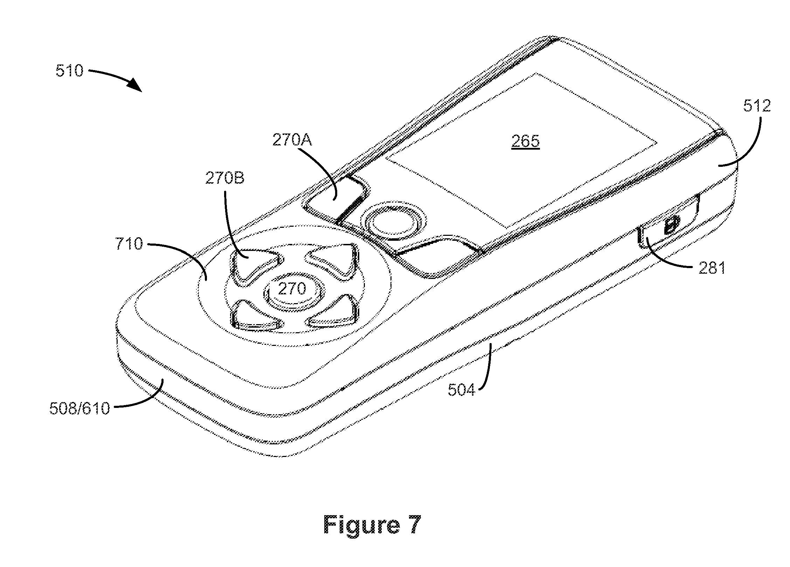

The completed external controller 510 is shown in FIG. 7. Notice that the chassis 508 (or sub-assembly 610 at this stage), as well as stabilizing the electrical components during manufacturing, also forms the outside edges of the external controller 510, and as such the case of the external controller 510 is made up of three pieces: the front cover 512, the chassis 508, and the back cover 504. Traditional external controllers typically only use front and back covers, which are bolted together in a clam-shell arrangement, and thus require the internal electronics to be somehow affixed to one of the front and back covers, or both. Because there is no need to affix the electronics to the front or back covers in the external controller 510, its design is simpler from this perspective. Moreover, because the external case of the external controller 510 comprises three different structures (the front cover 512, the chassis 508, and the back cover 504), its design is sturdy.

Although not shown, the back cover 504 could include a door for accessing the battery 126 in case it needs maintenance or changing. The back cover 504 could also include contacts to allow the battery 126 to be recharged while sitting in a charging cradle.

It should be noted that the front cover 512 has a recessed area 710. Placing some buttons 270A, but not other buttons 270B, into the recessed area 710 helps to differentiate the functionality of these buttons. For example, buttons 270A can provide basic external controller 510 functionality, and may include buttons for increasing and decreasing the level of stimulation, or for halting stimulation altogether. By contrast, buttons 270B can be relegated to controlling more advanced functions in the external controller 510, and may be used to navigate through the various menus provided by the user interface.

It should be understood that any use of the article "a" in this disclosure and the accompanying claims could mean one, or one or more.

Although particular embodiments of the present invention have been shown and described, it should be understood that the above discussion is not intended to limit the present invention to these embodiments.

## Claims

1. An external controller for an implantable medical device, comprising:
a chassis;
at least one telemetry coil affixed to the chassis, the at least one telemetry coil for communicating with an implantable medical device;
a circuit board affixed to the chassis;
a battery affixed to the chassis,
wherein the telemetry coil and the battery are electrically coupled to the circuit board;
a front cover placed proximate to a front side of the chassis; and
a back cover placed proximate to a back side of the chassis,
wherein the chassis comprises outside edges of the external controller between the front
and back covers; and wherein at least the chassis, the front cover, and the back cover further comprise at least one set of bolt holes for receiving a bolt to fasten the front cover, the back cover, and the chassis together.

2. The external controller of claim 1, further comprising at least one button integrated into the edges of the external controller.

3. The external controller of claim 1, wherein the circuit board is affixed to a first side of the chassis, and wherein the at least one telemetry coil and the battery are affixed to a second side of the chassis.

4. The external controller of claim 1, wherein the chassis further comprises a plurality of pins extending through first and second sides of the chassis, and wherein the at least one telemetry coil is electrically coupled to the pins on the first side of the chassis.

5. The external controller of claim 4, wherein the circuit board is electrically coupled to the pins on the second side of the chassis.

6. The external controller of claim 1, wherein the chassis further comprises clamps formed in the chassis, and wherein the circuit board is affixed within the chassis using the clamps.

7. The external controller of claim 1, wherein the chassis further comprises a recess for housing one of the at least one telemetry coil.

8. The external controller of claim 7, wherein the one telemetry coil is completely recessed below a top edge of the recess.

9. The external controller of claim 7, further comprising a ferrite core, wherein the one telemetry coil is wound around the ferrite core.

10. The external controller of claim 7, further comprising epoxy, wherein the recess is at least partially filled with the epoxy to affix the one telemetry coil.

11. The external controller of claim 1, wherein at least one telemetry coil affixed to the chassis comprises first and second telemetry coils, and wherein the first and second telemetry coils are wound around axes that are orthogonal.

12. The external controller of claim 11, wherein the chassis further comprises a first recess for the first telemetry coil and a second recess for the second telemetry coil.

13. The external controller of claim 1, wherein the chassis further comprises a recess for holding the battery.

14. The external controller of claim 13, further comprising an adhesive, wherein the battery is affixed in the recess using the adhesive.

15. The external controller of claim 1, wherein the battery further comprises a first connector, wherein the circuit board further comprises a second connector, wherein the chassis further comprises a hole, and wherein the battery electrically couples to the second connector through the hole.

16. A method of manufacturing the external controller of any one of claims 1-15 using a chassis comprising a plurality of pins, the method comprising:
forming a sub-assembly for the external controller, comprising affixing at least one telemetry coil to the chassis, the at least one telemetry coil for communicating with an implantable medical device;
electrically coupling the at least one telemetry coil to the pins;
affixing a circuit board to the chassis; and
electrically coupling the circuit board to the pins, thereby electrically coupling the at least one telemetry coil to the circuit board.

## Patentansprüche

1. Externe Steuereinrichtung für eine implantierbare medizinische Vorrichtung, die aufweist:
ein Chassis;
mindestens eine Telemetriespule, die am Chassis befestigt ist, wobei die mindestens eine Telemetriespule zum Kommunizieren mit einer implantierbaren medizinischen Vorrichtung dient;
eine Leiterplatte, die am Chassis befestigt ist;
eine Batterie, die am Chassis befestigt ist;
wobei die Telemetriespule und die Batterie elektrisch mit der Leiterplatte gekoppelt sind;
eine vordere Abdeckung, die nahe einer Vorderseite des Chassis angeordnet ist; und
eine hintere Abdeckung, die nahe einer Hinterseite des Chassis angeordnet ist,
wobei das Chassis zwischen der vorderen und hinteren Abdeckung Außenkanten der externen Steuereinrichtung aufweist; und wobei mindestens das Chassis, die vordere Abdeckung und die hintere Abdeckung ferner mindestens einen Satz von Bolzenlöchern zur Aufnahme eines Bolzens aufweisen, um die vordere Abdeckung, die hintere Abdeckung und das Chassis aneinander zu befestigen.

2. Externe Steuereinrichtung nach Anspruch 1, die ferner mindestens einen Knopf aufweist, der in die Kanten der externen Steuereinrichtung integriert ist.

3. Externe Steuereinrichtung nach Anspruch 1, wobei die Leiterplatte an einer ersten Seite des Chassis befestigt ist, und wobei die mindestens eine Telemetriespule und die Batterie an einer zweiten Seite des Chassis befestigt sind.

4. Externe Steuereinrichtung nach Anspruch 1, wobei das Chassis ferner mehrere Stifte aufweist, die sich durch die erste und zweite Seite des Chassis erstrecken, und wobei die mindestens eine Telemetriespule mit den Stiften auf der ersten Seite des Chassis elektrisch gekoppelt ist.

5. Externe Steuereinrichtung nach Anspruch 4, wobei die Leiterplatte auf der zweiten Seite des Chassis elektrisch mit den Stiften gekoppelt ist.

6. Externe Steuereinrichtung nach Anspruch 1, wobei das Chassis ferner Klammern aufweist, die im Chassis ausgebildet sind, und wobei die Leiterplatte innerhalb des Chassis mittels der Klammern befestigt ist.

7. Externe Steuereinrichtung nach Anspruch 1, wobei das Chassis ferner eine Aussparung zur Unterbringung von einer der mindestens einen Telemetriespule aufweist.

8. Externe Steuereinrichtung nach Anspruch 7, wobei die eine Telemetriespule unter einer Oberkante der Aussparung vollständig eingelassen ist.

9. Externe Steuereinrichtung nach Anspruch 7, die ferner einen Ferritkern aufweist, wobei die eine Telemetriespule um den Ferritkern gewickelt ist.

10. Externe Steuereinrichtung nach Anspruch 7, die ferner Epoxid aufweist, wobei die Aussparung mindestens teilweise mit dem Epoxid gefüllt ist, um die eine Telemetriespule zu befestigen.

11. Externe Steuereinrichtung nach Anspruch 1, wobei mindestens eine am Chassis befestigte Telemetriespule eine erste und zweite Telemetriespule aufweist, und wobei die erste und zweite Telemetriespule um Achsen gewickelt sind, die orthogonal sind.

12. Externe Steuereinrichtung nach Anspruch 11, wobei das Chassis ferner eine erste Aussparung für die erste Telemetriespule und eine zweite Aussparung für die zweite Telemetriespule aufweist.

13. Externe Steuereinrichtung nach Anspruch 1, wobei das Chassis ferner eine Aussparung zum Halten der Batterie aufweist.

14. Externe Steuereinrichtung nach Anspruch 13, die ferner ein Klebemittel aufweist, wobei die Batterie in der Aussparung mittels des Klebemittels befestigt ist.

15. Externe Steuereinrichtung nach Anspruch 1, wobei die Batterie ferner einen ersten Verbinder aufweist, wobei die Leiterplatte ferner einen zweiten Verbinder aufweist, wobei das Chassis ferner ein Loch aufweist, und wobei die Batterie durch das Loch mit dem zweiten Verbinder elektrisch gekoppelt ist.

16. Verfahren zum Herstellen der externen Steuereinrichtung nach einem der Ansprüche 1 bis 15 mittels eines Chassis, das mehrere Stifte aufweist, wobei das Verfahren aufweist: Bilden einer Baugruppe für die externe Steuereinrichtung, aufweisend:
Befestigen mindestens einer Telemetriespule an dem Chassis, wobei die mindestens eine Telemetriespule zum Kommunizieren mit einer implantierbaren medizinischen Vorrichtung dient;
elektrisches Koppeln der mindestens einen Telemetriespule mit den Stiften;
Befestigen einer Leiterplatte an dem Chassis; und
elektrisches Koppeln der Leiterplatte mit den Stiften, wodurch die mindestens eine Telemetriespule mit der Leiterplatte elektrisch gekoppelt wird.

## Revendications

1. Unité de commande externe pour un dispositif médical implantable, comprenant :
un châssis ;
au moins une bobine de télémesure fixée au châssis, ladite au moins une bobine de télémesure permettant de communiquer avec un dispositif médical implantable ;
une carte de circuit fixée au châssis ;
une pile fixée au châssis,
la bobine de télémesure et la pile étant électriquement reliées à la carte de circuit ;
un couvercle avant disposé à proximité d'une face avant du châssis ; et
un couvercle arrière disposé à proximité d'une face arrière du châssis,
le châssis comprenant les bords extérieurs de l'unité de commande externe entre le couvercle avant et le couvercle arrière ; et où au moins le châssis, le couvercle avant et le couvercle arrière comprennent en outre au moins un ensemble de trous pour boulons destinés à recevoir chacun un boulon pour fixer ensemble le couvercle avant, le couvercle arrière et le châssis.

2. Unité de commande externe selon la revendication 1, comprenant en outre au moins un bouton intégré aux bords de l'unité de commande externe.

3. Unité de commande externe selon la revendication 1, où la carte de circuit est fixée sur une première face du châssis, et où ladite au moins une bobine de télémesure et la pile sont fixées sur une deuxième face du châssis.

4. Unité de commande externe selon la revendication 1, où le châssis comprend en outre une pluralité de broches traversant la première et la deuxième faces du châssis, et où ladite au moins une bobine de télémesure est électriquement reliée aux broches sur la première face du châssis.

5. Unité de commande externe selon la revendication 4, où la carte de circuit est électriquement reliée aux broches sur la deuxième face du châssis.

6. Unité de commande externe selon la revendication 1, où le châssis comprend en outre des pinces formées dans le châssis, et où la carte de circuit est fixée à l'intérieur du châssis au moyen des pinces.

7. Unité de commande externe selon la revendication 1, où le châssis comprend en outre une cavité pour le logement d'une de ladite au moins une bobine de télémesure.

8. Unité de commande externe selon la revendication 7, où ladite une bobine de télémesure est complètement enfoncée sous un bord supérieur de la cavité.

9. Unité de commande externe selon la revendication 7, comprenant en outre un noyau en ferrite, ladite une bobine de télémesure étant enroulée autour du noyau en ferrite.

10. Unité de commande externe selon la revendication 7, comprenant en outre de la résine époxy, la cavité étant au moins partiellement remplie de résine époxy pour fixer ladite une bobine de télémesure.

11. Unité de commande externe selon la revendication 1, où au moins une bobine de télémesure fixée au châssis comprend une première et deuxième bobine de télémesures, et où la première et la deuxième bobines de télémesure sont enroulées autour d'axes orthogonaux.

12. Unité de commande externe selon la revendication 11, où le châssis comprend en outre une première cavité pour la première bobine de télémesure et une deuxième cavité pour la deuxième bobine de télémesure.

13. Unité de commande externe selon la revendication 1, où le châssis comprend en outre une cavité de maintien de la pile.

14. Unité de commande externe selon la revendication 13, comprenant en outre un adhésif, la pile étant fixée dans la cavité au moyen de l'adhésif.

15. Unité de commande externe selon la revendication 1, où la pile comprend en outre un premier connecteur, la carte de circuit comprenant en outre un deuxième connecteur, le châssis comprenant en outre un trou, et la pile étant électriquement reliée au deuxième connecteur par le trou.

16. Procédé de fabrication de l'unité de commande externe selon l'une des revendications 1 à 15 au moyen d'un châssis comprenant une pluralité de broches, ledit procédé comprenant :
la formation d'un sous-ensemble pour l'unité de commande externe, comprenant :
la fixation d'au moins une bobine de télémesure au châssis, ladite au moins une bobine de télémesure permettant de communiquer avec un dispositif médical implantable ;
la connexion électrique de ladite au moins une bobine de télémesure aux broches ;
la fixation d'une carte de circuit au châssis ; et
la connexion électrique de la carte de circuit aux broches, en reliant ainsi électriquement ladite au moins une bobine de télémesure à la carte de circuit.
